# EUROPÄISCHE PATENTSCHRIFT

(11) **EP 1 041 934 B1**
(45) Veröffentlichungstag und Bekanntmachung des Hinweises auf die Patenterteilung: **22.11.2006**
(21) Anmeldenummer: 99968765.0
(22) Anmeldetag: 27.10.1999
(51) Int. Cl.: A61B 17/32

(54) **MEDIZINISCHES INSTRUMENT ZUM ABTRAGEN VON GEWEBE**
MEDICAL INSTRUMENT FOR REMOVING TISSUE
INSTRUMENT MEDICAL POUR L'ENLEVEMENT DE TISSUS

(30) Priorität: 03.11.1998 DE 19850520
(43) Veröffentlichungstag der Anmeldung: 11.10.2000
(73) Patentinhaber: Karl Storz GmbH & Co. KG, 78532 Tuttlingen (DE)
(72) Erfinder: DORN, Jürgen, D-68809 Neulussheim (DE)
(74) Vertreter: Heuckeroth, Volker
(86) Internationale Anmeldenummer: PCT/EP1999/008101
(87) Internationale Veröffentlichungsnummer: WO 2000/025682

(56) Entgegenhaltungen:
- US-A- 5 320 635

## Beschreibung

Die Erfindung betrifft ein medizinisches Instrument zum Abtragen von Gewebe im menschlichen oder tierischen Körper, mit einem rohrförmigen Schaft, der im Bereich seines distalen Endes zumindest ein Fenster aufweist, mit einem Schneidelement, das in dem Schaft im Bereich des Fensters angeordnet und mit einer sich in dem Schaft erstreckenden Antriebswelle verbunden ist, über die das Schneidelement um seine Längsachse in Rotation antreibbar ist, wobei der Schaft zumindest eine Biegestelle aufweist, und wobei die Antriebswelle proximalseitig der Biegestelle endet und durch die Biegestelle hindurch über zumindest ein flexibles Element mit dem Schneidelement verbunden ist, wobei das zumindest eine flexible Element als Drahtelement ausgebildet ist, das an der Antriebswelle exzentrisch zu deren Längsmittelachse und/oder an dem Schneidelement exzentrisch zu dessen Längsmittelachse befestigt ist.

Ein derartiges Instrument ist aus der US-A-5 669 926 bekannt.

Ein solches Instrument, das auch als Rotationsschneidinstrument oder als Shaver bezeichnet wird, wird in der minimal-invasiven Chirurgie zum Abtragen von Gewebe im menschlichen oder tierischen Körper verwendet. Dazu wird das distale Ende des Schaftes durch eine Inzision in das Operationsgebiet geführt, in dem sich das abzutragende Gewebe befindet. Zum Abtragen des Gewebes wird das Schneidelement mittels eines externen oder internen Motors über die Antriebswelle in Rotation versetzt. Eine an dem Schneidelement ausgebildete Schneide wirkt während des Umlaufens mit einem ebenfalls als Schneide ausgebildeten Rand des Fensters des Schaftes schneidend zusammen, indem die Schneiden des Schneidelementes an den Schneiden des Fensters bei jedem Umlauf vorbeilaufen.

Neben Instrumenten, die einen durchgehend geraden Schaft aufweisen, sind auch Instrumente dieser Art bekannt, deren Schaft im Bereich des distalen Endes eine Biegestelle aufweist, d.h. der Schaft ist im Bereich seines distalen Endes gekrümmt. Durch die gekrümmte Ausgestaltung des Schafts können mit einem geraden Schaft nicht oder nur schwer zugängliche Gewebepartien im Körper abgetragen werden, bspw. in der Orthopädie können damit Gewebepartien um Gelenkstrukturen herum entfernt werden.

Bei einem Instrument mit einem gekrümmten Schaft stellt sich das Problem, die Rotation der an sich starren Antriebswelle durch die Biegestelle bzw. Krümmung des Schafts hindurch auf das Schneidelement zu übertragen, was deswegen problematisch ist, weil die Längsachse und damit Drehachse des Schneidelementes und die Längsachse und damit Drehachse der Antriebswelle einen von null verschiedenen Winkel einschließen.

Bei dem aus der eingangs genannten US-A-5 320 635 bekannten Instrument wird die Rotationsübertragung durch die Biegestelle hindurch dadurch bewerkstelligt, daß die als Rohrschaft ausgebildete Antriebswelle im in der Biegestelle angeordneten Bereich mit senkrecht zur Längsachse eingebrachten umfänglich begrenzten Einschnitten in der Art eines Balgs versehen ist, die eine Flexibilität der Antriebswelle in diesem Bereich ermöglichen. Es ist auch vorgesehen, daß die Antriebswelle zumindest in diesem Bereich aus einem flexiblen Kunststoff besteht.

An dieser Ausgestaltung ist jedoch nachteilig, daß die in die Antriebswelle eingebrachten Schnitte Sollbruchstellen darstellen können, da die Antriebswelle im Biegebereich beim Umlaufen ständig wechselnden Biegerichtungen ausgesetzt ist. Außerdem ist diese Ausgestaltung der Antriebswelle, insbesondere bei einer miniaturisierten Ausgestaltung des Instruments mit einem dünnen Schaft hinsichtlich Herstellung und Kosten aufwendig, da die Schnitte sehr fein ausgebildet werden müssen, wozu geeignete Werkzeuge verwendet werden müssen.

Ein weiteres Rotationsschneidinstrument ist aus der DE-A-43 23 756 bekannt. Bei diesem Instrument ist die Antriebswelle im Bereich der Biegestelle des Schafts unterbrochen, wobei die jeweils benachbarten Enden der starren Wellenteile über ein oder mehrere Kreuzgelenke in Verbindung stehen. Die Kreuzgelenke weisen zwei zueinander orthogonale Gelenkachsen auf.

Diese bekannte Art der Rotationsübertragung von der Antriebswelle auf das Schneidelement führt ebenfalls zu einem komplizierten Aufbau des Instrumentes. Insbesondere bei miniaturisierten Ausgestaltungen des Instrumentes müssen die Kreuzgelenke ebenfalls miniaturisiert ausgebildet sein. Die Herstellung solcher miniaturisierter Kreuzgelenke ist jedoch aufwendig.

Aus der US 5,669,926 ist weiterhin ein Rotationsschneidinstrument bekannt, bei dem die Antriebswelle durch eine Biegestelle des Rohrschaftes hindurch über eine flexible Wendel mit dem Schneidelement verbunden ist, wobei die Wendel den gleichen Durchmesser aufweist wie die Antriebswelle. Auch diese Art der Rotationsübertragung durch eine Biegestelle hindurch ist mit einem erhöhten Aufwand bei der Herstellung der Antriebswelle verbunden.

Aus der DE 43 02 912 A1 ist ein Rotationsschneidinstrument bekannt, wobei die Antriebswelle in einem Krümmungsbereich des Rohrschafts aus Kardanelementen aufgebaut ist.

Eine ähnliche Ausgestaltung der Antriebswelle im Bereich einer Biegung des Rohrschafts durch eine Hintereinanderreihung miteinander in Eingriff stehender Kardanelemente ist aus der US 5,755,731 bekannt.

Eine der Ausgestaltung der Antriebswelle gemäß der eingangs genannten US 5,320,635 ähnliche Antriebswelle ist bei einem Rotationsschneidinstrument aus der US 5,620,447 bekannt.

Schließlich ist aus der US 5,529,580 ein Rotationsschneidinstrument bekannt, bei dem die Rotationsübertragung von der Antriebswelle durch eine Biegung des Rohrschafts hindurch auf das Schneidelement durch ein schraubenfederartiges Element erfolgt.

Alle zuvor genannten bekannten Arten der flexiblen Ausgestaltung von der Antriebswelle im Bereich der Biegung des Schafts haben den Nachteil, konstruktiv und herstellungstechnisch aufwendig zu sein.

Der Erfindung liegt daher die Aufgabe zugrunde, ein Instrument der eingangs genannten Art dahingehend weiterzubilden, daß eine Rotationsübertragung von der Antriebswelle auf das Schneidelement durch die zumindest eine Biegestelle mit geringem konstruktivem Aufwand ermöglicht wird.

Erfindungsgemäß wird diese Aufgabe hinsichtlich des eingangs genannten Instrumentes dadurch gelöst, daß der Durchmesser des Drahtelements kleiner ist als der Durchmesser der Antriebswelle.

Anstelle der im Stand der Technik vorgesehenen Rotationsübertragung von der Antriebswelle auf das Schneidelement mittels einer mit Einschnitten versehenen Ausgestaltung der Antriebswelle oder mittels Kreuzgelenken wird bei dem erfindungsgemäßen Instrument demnach die Rotationsübertragung mittels zumindest eines flexiblen Drahtelementes bewerkstelligt, das die Antriebswelle mit dem Schneidelement verbindet. Eine solche Verbindung mittels zumindest eines flexiblen Drahtelementes ist konstruktiv sehr einfach, so daß das erfindungsgemäße Instrument mit geringem technischen Aufwand kostengünstig herstellbar ist. Durch die exzentrische Befestigung der beiden Drahtelementenden an der Antriebswelle einerseits und dem Schneidelement andererseits wird zudem eine Rotationsübertragung mit einer günstigen Drehmomentübertragung erreicht. Das flexible Drahtelement hat weiterhin den Vorteil, daß es sich an den gekrümmten Verlauf der Biegestelle des Schafts anpaßt und somit für jeden Krümmungsradius der Biegestelle geeignet ist. Das Drahtelement kann dabei an der Antriebswelle und dem Schneidelement an der jeweils gleichen Winkelposition, d.h. ohne umfänglichen Versatz oder an bezüglich der Antriebswelle und dem Schneidelement unterschiedlichen Winkelpositionen befestigt sein.

Die exzentrisch zur Längsmittelachse der Antriebswelle und exzentrisch zur Längsmittelachse des Schneidelementes vorgesehene Befestigung des Drahtelementes eröffnet weiterhin die besonders vorteilhafte Möglichkeit, die Schneidwirkung bzw. -leistung des erfindungsgemäßen Instrumentes wie nachstehend erläutert zu erhöhen. Dies kann bspw. dann, wenn der Schaft nur eine Biegestelle aufweist, dadurch erreicht werden, daß das Drahtelement an der Antriebswelle und an dem Schneidelement exzentrisch ohne umfänglichen Versatz befestigt wird. Beim Rotieren des Drahtelementes um die Längsmittelachse führt dann das Schneidelement eine Drehung um seine Längsmittelachse aus, der eine hin- und hergehende geringfügige Translationsbewegung überlagert ist. Der Hub der Translationsbewegung entspricht dabei der Differenz aus der Außenbogenlänge und der Innenbogenlänge der Wand des Schafts im Bereich der Biegestelle. Durch die sich überlagernde Rotations- und Translationsbewegung des Schneidelementes wird die Schneidwirkung des Schneidelementes beim Umlaufen in dem Fenster des Schafts verbessert, da die Schneidenbewegung nicht nur eine Komponente orthogonal zur Schneide, sondern auch in Längsrichtung der Schneide aufweist, wodurch ein ziehender Schnitt in das Gewebe eingebracht wird. Grundsätzlich kann aber das Drahtelement an beliebigen Winkelpositionen an der Antriebswelle und an dem Schneidelement befestigt sein.

Unter Drahtelement im Sinne der vorliegenden Erfindung ist nicht nur ein Draht aus Vollmaterial, sondern auch ein länglicher, hohler Körper zu verstehen.

Somit wird die der Erfindung zugrundeliegende Aufgabe vollkommen gelöst.

In einer bevorzugten Ausgestaltung ist das Drahtelement am Umfang der Antriebswelle und/oder am Umfang des Schneidelements befestigt.

Bei dieser Ausgestaltung wird vorteilhafterweise eine maximale Drehmomentübertragung von der Antriebswelle auf das Schneidelement zum Antreiben des Schneidelements in Rotation bewirkt. Außerdem kann der zuvor erwähnte Effekt der dem Schneidelement zusätzlich verliehenen Translationsbewegung in Richtung seiner Längsmittelachse durch diese Ausgestaltung verstärkt werden.

Dabei ist es bevorzugt, wenn das Drahtelement im Bereich der Befestigung an der Antriebswelle und/oder im Bereich der Befestigung an dem Schneidelement zur Längsmittelachse des Schafts hin abgekröpft ist.

Diese Ausgestaltung hat den Vorteil, daß das Drahtelement zwischen der Antriebswelle und dem Schneidelement zur Längsmittelachse des Schafts hin verlagert wird, wodurch eine Reibung des Drahtelementes beim Umlaufen in dem Schaft vermindert wird.

In einer weiteren bevorzugten Ausgestaltung weist der Schaft genau eine Biegestelle auf, und ist das Drahtelement an der Antriebswelle und an dem Schneidelement ohne umfänglichen Versatz befestigt.

Bei dieser Ausgestaltung des erfindungsgemäßen Instruments wird die bereits zuvor erläuterte, dem Schneidelement zusätzlich zu seiner Rotationsbewegung auferlegte hin- und hergehende Translationsbewegung bei einem Schaft mit nur einer Biegestelle erreicht.

In einer alternativen Ausgestaltung weist der Schaft zwei zueinander gegensinnige Biegestellen auf, und ist das Drahtelement an der Antriebswelle und an dem Schneidelement ohne umfänglichen Versatz befestigt.

Diese Ausgestaltung ist vorteilhaft, wenn eine Translationsbewegung des Schneidelements beim Rotieren des Schneidelements unterdrückt werden soll. Bei Befestigung des Drahtelements am Schneidelement und an der Antriebswelle in gleicher Winkelposition zueinander heben sich die Weglängendifferenzen aus Außenbogenlänge und Innenbogenlänge der ersten Biegestelle und Außenbogenlänge und Innenbogenlänge der zweiten Biegestelle nahezu gegeneinander auf, wenn das Drahtelement in dem Schaft umläuft. Eine exakte Aufhebung dieser Weglängendifferenzen erfolgt dabei dann, wenn die Krümmungsradien der beiden Biegestellen gleich groß sind.

In einer weiteren bevorzugten alternativen Ausgestaltung weist der Schaft zwei zueinander gegensinnige Biegestellen auf, und ist das Drahtelement an der Antriebswelle und an dem Schneidelement an einer zueinander um etwa 180° versetzten Winkelposition befestigt.

Bei einer Ausgestaltung des Schafts mit zwei Biegestellen, die gegensinnig zueinander sind, so daß der Schaft einen etwa Z-förmigen Verlauf aufweist, kann die erwähnte Translationsbewegung des Schneidelementes durch eine um 180° versetzte Befestigung des Drahtelementes an der Antriebswelle einerseits und dem Schneidelement andererseits erreicht werden. Bei dieser Ausgestaltung wird die Translationsbewegung des Schneidelementes sogar noch verstärkt, da sich hier die Weglängendifferenzen aus Außenbogenlänge und Innenbogenlänge der ersten Biegestelle und Außenbogenlänge und Innenbogenlänge der zweiten Biegestelle zueinander addieren. Der Hub der Translationsbewegung kann somit gegenüber dem Hub der Translationsbewegung bei der Ausgestaltung des Schafts mit nur einer Biegestelle weiter verstärkt werden. Die erfindungsgemäße Ausgestaltung des Instrumentes eignet sich insbesondere auch dann, wenn der Schaft beliebig viele Biegestellen aufweist, so daß eine Rotationsübertragung von der Antriebswelle auf das Schneidelement bei beliebig gebogenen Ausgestaltungen des Schafts bewerkstelligt werden kann.

In einer weiteren bevorzugten Ausgestaltung ist das Drahtelement ein Draht aus Vollmaterial oder ein Rohr, dessen Durchmesser kleiner als der halbe Innendurchmesser des Schafts ist.

Hierdurch werden konstruktiv vorteilhaft einfache und kostengünstige Ausgestaltungen des zumindest einen Drahtelements geschaffen.

In einer weiteren bevorzugten Ausgestaltung ist das Drahtelement aus Federstahl.

Hierbei ist von Vorteil, daß das Drahtelement eine genügend hohe Flexibilität besitzt, um sich an den Verlauf des Schafts im Bereich der Biegestelle anpassen zu können. Außerdem erhält das Drahtelement eine genügend hohe Elastizität, so daß es den beim Umlaufen ständig wechselnden Biegebeanspruchungen auch bei hohen Drehzahlen auf Dauer standhalten kann.

Vorteilhafterweise ist das Drahtelement an der Antriebswelle und/oder an dem Schneidelement durch Löten, Schweißen, Kleben oder dgl. befestigt.

Diese Befestigungsarten sind vorteilhaft einfach und reduzieren weiterhin den Herstellungs- und Kostenaufwand des erfindungsgemäßen Instruments.

In einer weiteren bevorzugten Ausgestaltung ist das Drahtelement mit seinem proximalen Ende in die Antriebswelle und/oder mit seinem distalen Ende in das Schneidelement eingesteckt.

Hierbei ist von Vorteil, daß durch das Einstecken des Drahtelementes in das Schneidelement bzw. in die Antriebswelle ein bezüglich Tangentialkräften bzw. Scherkräften sicher haftender Verbund zwischen dem Drahtelement und dem Schneidelement bzw. zwischen dem Drahtelement und der Antriebswelle erreicht wird.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein medizinisches Instrument zum Abtragen von Gewebe in Seitenansicht;
- Fig. 2: einen Längsschnitt durch das Instrument in Fig. 1 bei abgenommenem Handgriff;
- Fig. 3: einen Längsschnitt durch die Anordnung aus Antriebswelle, Drahtelement und Schneidelement in Alleinstellung;
- Fig. 4: einen Längsschnitt durch den Schaft des Instruments in Fig. 1 in Alleinstellung; und
- Fig. 5: einen Längsschnitt durch den distalen Bereich eines Instruments zum Abtragen von Gewebe gemäß einem weiteren Ausführungsbeispiel.

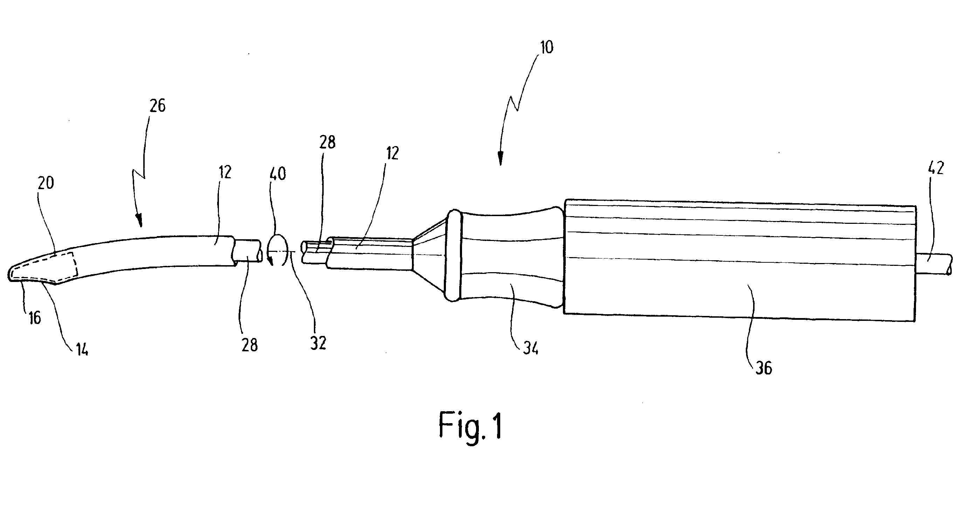

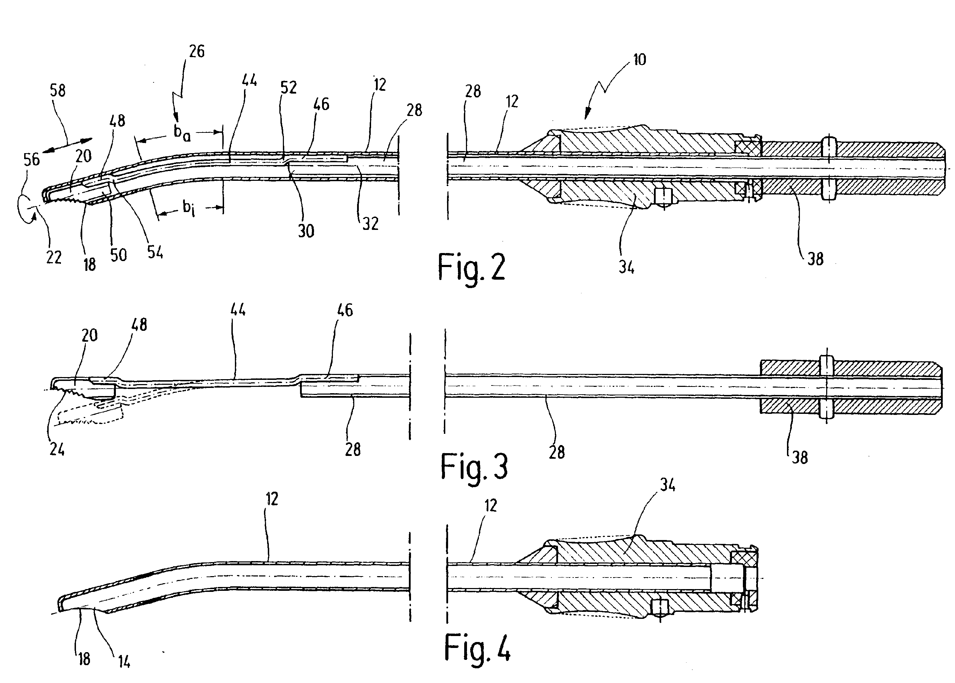

In Fig. 1 ist ein mit dem allgemeinen Bezugszeichen 10 versehenes medizinisches Instrument zum Abtragen von Gewebe im menschlichen oder tierischen Körper dargestellt. Teile des Instruments 10 sind weiterhin in Fig. 2 bis 4 dargestellt.

Das Instrument 10 weist einen langerstreckten Schaft 12 auf. Der Schaft 12 ist als Rohr ausgebildet. Im Bereich seines distalen Endes weist der Schaft 12 ein Fenster 14 auf. Das Fenster 14 erstreckt sich dabei über einen Teilumfangsbereich des Schafts 12. Das Fenster 14 stellt eine Öffnung in dem Schaft 12 dar, dessen seitliche Ränder 16 (Fig. 1) und 18 (Fig. 4) als Schneidkanten ausgebildet sind.

Im Bereich des Fensters 14 ist in dem Schaft 12 ein Schneidelement 20 angeordnet. Das Schneidelement 20 ist in Form eines zylindrischen Hohlkörpers ausgebildet, dessen Außenkontur an die Innenkontur des Schafts 12 angepaßt ist, so daß das Schneidelement 20 formschlüssig im distalen Ende des Schaftes 12 aufgenommen ist.

Das Schneidelement 20 ist in dem Schaft 12 um seine Längsmittelachse 22 drehbar in dem Schaft 12 an dessen Innenwand gelagert. Der Schaft 12 selbst ist drehfest ausgebildet.

Das Schneidelement 20 weist ferner zwei seitliche Schneidkanten 24 auf, die beim Rotieren des Schneidelements 20 in dem Schaft 12 an den Rändern 16 und 18 des Fensters 14 vorbeilaufen und mit diesen dann zum Abtragen von Gewebe schneidend zusammenwirken. Die Schneidkanten 24 sind, wie aus Fig. 3 hervorgeht, gezahnt ausgeführt.

Der Schaft 12 weist ferner eine Biegestelle 26 auf, an der der Schaft 12 eine Krümmung aufweist. Das Schneidelement 20 ist dabei distalseitig von der Biegestelle 26 in einem geraden Abschnitt des Schafts 12 angeordnet.

Um das Schneidelement 20 zum Abtragen von Gewebe in Rotation zu versetzen, ist das Schneidelement 20 mit einer Antriebswelle 28 verbunden. Die Antriebswelle 28 ist wie der Schaft 12 im Querschnitt rund. In dem gezeigten Ausführungsbeispiel ist die Antriebswelle 28 als hohlzylindrisches Rohr ausgebildet. Die Antriebswelle 28 kann jedoch auch als massiver Stab ausgebildet sein.

Ein distales Ende 30 der Antriebswelle 28 endet proximalseitig von der Biegestelle 26 des Schafts 12.

Die Antriebswelle 28 ist ferner starr ausgebildet. Die Antriebswelle 28 ist um ihre Längsmittelachse 32 drehbar in dem feststehenden Schaft 12 aufgenommen.

Der Schaft 12 ist weiterhin über eine Kupplung 34 drehfest mit einem Handgriffgehäuse 36 verbunden, das nur in Fig. 1 dargestellt ist.

Am proximalen Ende der Antriebswelle 28 ist eine weitere Kupplung 38 drehfest mit dieser verbunden, die ebenfalls in dem Handgriffgehäuse 36 in Fig. 1 angeordnet ist.

In dem Handgriffgehäuse 36 ist weiterhin ein nicht dargestellter Antriebsmotor angeordnet, der mit der Antriebswelle 28 verbunden ist, um diese in Rotation gemäß einem Pfeil 40 in Fig. 1 anzutreiben. Der Motor wird über ein Stromzuführungskabel 42 am proximalen Ende des Handgriffgehäuses 36 von einer externen Spannungsquelle mit Strom beaufschlagt.

Wie aus Fig. 2 hervorgeht, bilden die Längsmittelachse 22 des Schneidelementes 20 und die Längsmittelachse 32 der Antriebswelle 28 aufgrund der Biegestelle 26 des Schafts 12 einen Winkel miteinander. Um die Rotation der Antriebswelle 28 auf das Schneidelement 20 übertragen zu können, ist das Schneidelement 20 mit der Antriebswelle 28 über zumindest ein flexibles Drahtelement 44 durch die Biegestelle 26 hindurch verbunden.

Das Drahtelement 44 ist mit seinem proximalen Ende 46 mit dem distalen Ende 30 der Antriebswelle 28 exzentrisch zu deren Längsmittelachse 32 befestigt.

Ein distales Ende 48 des Drahtelementes 44 ist an einem proximalen Ende 50 des Schneidelementes 20 ebenfalls exzentrisch zu dessen Längsmittelachse 22 befestigt. Dabei ist das proximale Ende 46 wie das distale Ende 48 des Drahtelementes 44 genauer gesagt am Umfang der Antriebswelle 28 bzw. am Umfang des Schneidelementes 20 mit der maximal möglichen Exzentrizität befestigt.

Weiterhin geht aus Fig. 2 und 3 hervor, daß das Drahtelement 44 an der Antriebswelle 28 und an dem Schneidelement 20 ohne umfänglichen Versatz zwischen beiden Befestigungsstellen befestigt ist.

In einem Bereich 52 der Befestigung des Drahtelementes 44 an der Antriebswelle 28 und in einem Bereich 54 der Befestigung des Drahtelementes 44 an dem Schneidelement 20 ist das Drahtelement 44 zur Längsmittelachse des Schafts 12 hin abgekröpft. Beim Umlaufen des Drahtelementes 44 in dem Schaft 12 wird dadurch eine Reibung des Drahtelementes 44 an dem Schaft 12 vermieden. Die Abkröpfung in den Bereichen 52 und 54 ist dabei gemäß Fig. 2 so weit ausgeprägt, daß das Drahtelement 44 in dem Schaft 12 noch außerhalb der Längsmittelachse des Schafts 12 verläuft.

Die Abkröpfung in den Bereichen 52 und 54 kann jedoch auch so stark ausgeprägt sein, daß das Drahtelement 44 im wesentlichen auf der gekrümmt verlaufenden Längsmittelachse des Schafts 12 im Bereich der Biegestelle 26 liegt.

Das Drahtelement 44 ist als Draht aus Vollmaterial oder als dünnes flexibles Rohr ausgebildet. Im Falle der Ausgestaltung des Drahtelementes 44 als Rohr ist der Durchmesser des Rohres dann kleiner als der halbe Innendurchmesser des Schafts 12.

Bevorzugt ist das Drahtelement 44 aus Federstahl gefertigt.

Wie aus Fig. 3 hervorgeht, in der die Anordnung aus der Antriebswelle 28, dem Drahtelement 44 und dem Schneidelement 20 in Alleinstellung dargestellt ist, nimmt das Drahtelement 44, wenn keine Biegekräfte auf dieses wirken, eine geradlinige Form ein. Durch diese flexible und im Ruhezustand des Drahtelementes 44 gerade Ausgestaltung des Drahtelementes 44 ist dieses geeignet, sich an jedem beliebigen Krümmungsverlauf der Biegestelle 26, auch an Mehrfachbiegungen des Schafts 12 anzupassen.

Die Befestigung des Drahtelementes 44 an der Antriebswelle 28 und an dem Schneidelement 20 kann durch Schweißen, Löten, Kleben oder dgl. bewerkstelligt werden. Das Drahtelement 44 kann außerdem in die Antriebswelle 28 bzw. in das Schneidelement 20 eingesteckt sein, wofür das distale Ende 30 der Antriebswelle 28 bzw. das proximale Ende 50 des Schneidelementes 20 einen längsverlaufenden Schlitz aufweist, in die das proximale Ende 46 bzw. das distale Ende 48 des Drahtelementes 44 eingeschoben ist.

Durch das Drahtelement 44 wird eine Rotation der Antriebswelle 28 gemäß dem Pfeil 40 in Fig. 1 in eine entsprechende Rotation des Schneidelementes 20 gemäß einem Pfeil 56 in Fig. 2 übertragen. Die Rotationsübertragung durch das Drahtelement 44 erfolgt dabei exzentrisch zur Längsmittelachse des Schafts 12.

Aufgrund dieser exzentrischen Rotationsübertragung stellt sich darüber hinaus folgender Effekt ein. Bei der in Fig. 2 dargestellten Drehstellung des Drahtelementes 44 liegt dieses im Bereich der Biegestelle 26 demjenigen Wandabschnitt des Schafts 12 gegenüber eine Außenbogenlänge bₐ aufweist. Beim weiteren Umlaufen des Drahtelementes 44 um 180° liegt das Drahtelement 44 demjenigen Wandabschnitt des Schafts 12 gegenüber, der eine Innenbogenlänge bᵢ aufweist. Da die Außenbogenlänge bₐ größer ist als die Innenbogenlänge bᵢ, die Länge des Drahtelementes 44 jedoch unveränderlich ist, bewirkt die exzentrische Anordnung des Drahtelementes 44 beim Umlaufen eine Hin- und Herverschiebung des Schneidelementes 20 in Richtung seiner Längsachse 22 gemäß einem Doppelpfeil 58.

Das Schneidelement 20 führt somit eine Rotationsbewegung aus, die von einer Translationsbewegung überlagert ist. Der Hub der Translationsbewegung entspricht dabei der Differenz zwischen, der Außenbogenlänge bₐ und der Innenbogenlänge bᵢ. Da diese Differenz gering ist, kann die Translationsbewegung des Schneidelements 20 auch als Mikrotranslation bezeichnet werden. Die Überlagerung aus Rotationsbewegung und oszillierender Translationsbewegung bewirkt eine erhöhte Schneideffizienz des Schneidelements 20.

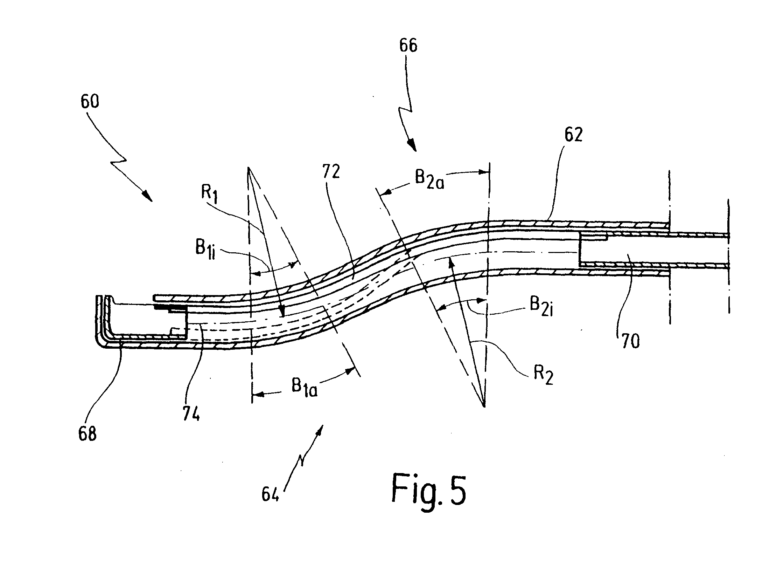

In Fig. 5 ist nun ein weiteres Ausführungsbeispiel eines mit dem allgemeinen Bezugszeichen 60 versehenen Instruments dargestellt, wobei das Instrument 60 lediglich im Bereich seines distalen Endes dargestellt ist.

Ein Schaft 62 des Instruments 60 weist zwei Biegestellen auf, und zwar eine erste Biegestelle 64 und eine zweite Biegestelle 66. Die erste Biegestelle 64 und die zweite Biegestelle 66 sind gegensinnig.

Ein Schneidelement 68 ist mit einer Antriebswelle 70 über ein Drahtelement 72 verbunden. Wie bei dem vorherigen Ausführungsbeispiel ist das Drahtelement 72 mit der Antriebswelle 70 und mit dem Schneidelement 68 ohne umfänglichen Versatz verbunden.

Wenn ein Krümmungsradius R₁ einer Längsmittelachse 74 des Schafts 62 an der ersten Biegestelle 64 und ein Krümmungsradius R₂ der Längsmittelachse 74 des Schafts 62 im Bereich der zweiten Biegestelle 66 gleich groß sind, wird bei dieser Anordnung des Drahtelementes 72 die zuvor in bezug auf das Schneidelement 20 beschriebene Translationsbewegung des Schneidelements 68 beim Rotieren in dem Schaft 62 unterdrückt.

In diesem Fall kompensieren sich nämlich bei gleichen Krümmungsradien R₁ und R₂ die Weglängendifferenzen aus einer Außenbogenlänge B₁ₐ und einer Innenbogenlänge B₁ᵢ an der ersten Biegestelle 64 und die Weglängendifferenz aus einer Außenbogenlänge B₂ₐ und einer Innenbogenlänge B₂ᵢ an der zweiten Biegestelle 66 insgesamt zu null, weil das Drahtelement 72 beim Umlaufen an der ersten Biegestelle 64 entweder an einem Wandabschnitt des Schafts 62 mit der kleineren Innenbogenlänge B₁ᵢ und an der zweiten Biegestelle 66 einem Wandabschnitt des Schafts 62 mit der größeren Außenbogenlänge B₂ₐ gegenüberliegt oder bei einer um 180° verdrehten Drehstellung umgekehrt.

Bei der Ausgestaltung des Schafts 62 mit einer doppelten Biegung wie in Fig. 5 kann jedoch auch eine Translationsbewegung des Schneidelements 68 beim Umlaufen in dem Schaft 62 erreicht werden, wenn das Drahtelement 72 an der Antriebswelle 70 und an dem Schneidelement 68 in um etwa 180° versetzter Winkellage befestigt ist, wie mit unterbrochenen Linien alternativ dargestellt ist. In diesem Fall kompensieren sich die zuvor genannten Weglängendifferenzen (B₁ₐ - B₁ᵢ) und (B₂ₐ - B₂ᵢ) nicht, sondern, im Gegenteil, addieren sich. Der Hub der Translationsbewegung des Schneidelementes 68 kann somit gegenüber dem in Fig. 1 bis 4 dargestellten Ausführungsbeispiel, bei dem der Schaft 12 nur eine Biegestelle aufweist, sogar noch vergrößert werden.

Bei dem in Fig. 1 bis 4 dargestellten Ausführungsbeispiel kann es ebenso vorgesehen sein, das Drahtelement 44 an dem Schneidelement 20 und an der Antriebswelle 32 um 180° versetzt zu befestigen. Das Drahtelement 44 und das Drahtelement 72 können auch an beliebig zueinander versetzten Befestigungsstellen an der Antriebswelle einerseits und dem Schneidelement andererseits befestigt sein. Während in den Figuren nur ein Drahtelement dargestellt ist, können im Rahmen der Erfindung auch zwei oder mehrere Drahtelemente zur Rotationsübertragung verwendet werden.

## Patentansprüche

1. Medizinisches Instrument zum Abtragen von Gewebe im menschlichen oder tierischen Körper, mit einem rohrförmigen Schaft (12; 62), der im Bereich seines distalen Endes zumindest ein Fenster (14) aufweist, mit einem Schneidelement (20; 68), das in dem Schaft (12; 62) im Bereich des Fensters (14) angeordnet und mit einer sich in dem Schaft (12; 62) erstreckenden Antriebswelle (28; 70) verbunden ist, über die das Schneidelement (20; 68) um seine Längsachse (22) in Rotation antreibbar ist, wobei der Schaft (12; 62) zumindest eine Biegestelle (26; 64, 66) aufweist, und wobei die Antriebswelle (28; 70) proximalseitig der Biegestelle (26; 64, 66) endet und durch die Biegestelle (26; 64, 66) hindurch über zumindest ein flexibles Element mit dem Schneidelement (20; 68) verbunden ist, wobei das zumindest eine flexible Element als Drahtelement (44; 72) ausgebildet ist, das an der Antriebswelle (28; 70) exzentrisch zu deren Längsmittelachse (32) und/oder an dem Schneidelement (20; 68) exzentrisch zu dessen Längsmittelachse (22) befestigt ist, **dadurch gekennzeichnet, daß** der Durchmesser des Drahtelements kleiner ist als der Durchmesser der Antriebswelle (28; 70).

2. Instrument nach Anspruch 1, **dadurch gekennzeichnet, daß** das Drahtelement (44, 72) am Umfang der Antriebswelle (28; 70) und/oder am Umfang des Schneidelements (20; 68) befestigt ist.

3. Instrument nach Anspruch 2, **dadurch gekennzeichnet, daß** das Drahtelement (44) im Bereich der Befestigung an der Antriebswelle (28) und/oder im Bereich der Befestigung an dem Schneidelement (20) zur Längsmittelachse des Schafts (12) hin abgekröpft ist.

4. Instrument nach einem der Ansprüche 1 bis 3, **dadurch gekennzeichnet, daß** der Schaft (12) genau eine Biegestelle (26) aufweist, und daß das Drahtelement (44) an der Antriebswelle (28) und an dem Schneidelement (20) ohne umfänglichen Versatz befestigt ist.

5. Instrument nach einem der Ansprüche 1 bis 3, **dadurch gekennzeichnet, daß** der Schaft (62) zwei zueinander gegensinnige Biegestellen (64, 66) aufweist, und daß das Drahtelement (72) an der Antriebswelle (70) und an dem Schneidelement (72) ohne umfänglichen Versatz befestigt ist.

6. Instrument nach einem der Ansprüche 1 bis 3, **dadurch gekennzeichnet, daß** der Schaft (62) zwei zueinander gegensinnige Biegestellen (64, 66) aufweist, und daß das Drahtelement (72) an der Antriebswelle (70) und an dem Schneidelement (68) an einer zueinander um etwa 180° versetzten Winkelposition befestigt ist.

7. Instrument nach einem der Ansprüche 1 bis 6, **dadurch gekennzeichnet, daß** das Drahtelement (44; 72) ein Draht aus Vollmaterial oder ein Rohr ist, dessen Durchmesser kleiner als der halbe Innendurchmesser des Schafts (12; 62) ist.

8. Instrument nach einem der Ansprüche 1 bis 7, **dadurch gekennzeichnet, daß** das Drahtelement (44; 72) aus Federstahl ist.

9. Instrument nach einem der Ansprüche 1 bis 8, **dadurch gekennzeichnet, daß** das Drahtelement (44; 72) an der Antriebswelle (28; 70) und/oder an dem Schneidelement (20; 68) durch Löten, Schweißen oder Kleben befestigt ist.

10. Instrument nach einem der Ansprüche 1 bis 9, **dadurch gekennzeichnet, daß** das Drahtelement (44; 72) mit seinem proximalen Ende (46) in die Antriebswelle (28; 70) und/oder mit seinem distalen Ende (48) in das Schneidelement (20) eingesteckt ist.

## Claims

1. A medical instrument for removing tissue in the human or animal body, comprising a tubular shaft (12; 62) that has at least one window (14) in the region of its distal end, a cutting element (20; 68) that is arranged in the shaft (12; 62) in the region of the window (14) and is connected to a drive shaft (28; 70), extending in the shaft (12; 62), by way of which the cutting element (20; 68) can be driven rotationally about its longitudinal axis (22), the shaft (12; 62) having at least one bending point (26; 64, 66), and the drive shaft (28; 70) terminating proximally from the bending point (26; 64, 66) and being connected to the cutting element (20; 68) through the bending point (26; 64, 66) by way of at least one flexible element, wherein the at least one flexible element is configured as a wire element (44; 72), which is attached to the drive shaft (28; 70) eccentrically with respect to the latter's longitudinal center axis (32) and/or to the cutting element (20; 68) eccentrically with respect to the latter's longitudinal center axis (22), **characterized in that** the diameter of the wire element is less than the diameter of the drive shaft (28; 70).

2. The instrument of claim 1, **characterized in that** the wire element (44; 72) is attached at the circumference of the drive shaft (28; 70) and/or at the circumference of the cutting element (20; 68).

3. The instrument of claim 2, **characterized in that** the wire element (44), in the region of the attachment to the drive shaft (28) and/or in the region of the attachment to the cutting element (20), is cranked toward the longitudinal center axis of the shaft (12).

4. The instrument of anyone of claims 1 through 3, **characterized in that** the shaft (12) has exactly one bending point (26), and the wire element (44) is attached to the drive shaft (28) and to the cutting element (20) without circumferential offset.

5. The instrument of anyone of claims 1 through 3, **characterized in that** the shaft (62) has two bending points (64, 66) directed oppositely from one another; and the wire element (72) is attached to the drive shaft (70) and to the cutting element (68) without circumferential offset.

6. The instrument of anyone of claims 1 through 3, **characterized in that** the shaft (62) has two bending points (64, 66) directed oppositely from one another; and the wire element (72) is attached to the drive shaft (70) and to the cutting element (68) at angular positions offset approximately 180° from one another.

7. The instrument of anyone of claims 1 through 6, **characterized in that** the wire element (44; 72) is a wire made of solid material or a tube whose diameter is less than half the inside diameter of the shaft (12; 62).

8. The instrument of anyone of claims 1 through 7, **characterized in that** the wire element (44; 72) is made of spring steel.

9. The instrument of anyone of claims 1 through 8, **characterized in that** the wire element (44; 72) is attached to the drive shaft (28; 70) and/or to the cutting element (20; 68) by soldering, welding, or adhesive bonding.

10. The instrument of anyone of claims 1 through 9, **characterized in that** the wire element (44; 72) is inserted with its proximal end (46) into the drive shaft (28; 70) and/or with its distal end (48) into the cutting element (20).

## Revendications

1. Instrument médical pour l'ablation de tissus dans le corps humain ou animal, comprenant une tige (12 ; 62) tubulaire, qui comporte au moins une fenêtre (14) dans la zone de son extrémité distale, comprenant un élément de coupe (20 ; 68), qui est disposé dans la tige (12 ; 62) dans la zone de la fenêtre (14) et est relié à un arbre d'entraînement (28 ; 70), qui s'étend dans la tige (12 ; 62) et par l'intermédiaire duquel l'élément de coupe (20 ; 68) est entraîné en rotation autour de son axe longitudinal (22), la tige (12 ; 62) comportant au moins une zone de flexion (26 ; 64, 66) et l'arbre d'entraînement (28 ; 70) se terminant du côté proximal de la zone de flexion (26 ; 64, 66) et étant relié en passant par la zone de flexion (26 ; 64, 66) à l'élément de coupe (20 ; 68) par l'intermédiaire d'au moins un élément flexible, ledit au moins un élément flexible étant réalisé sous forme d'élément filaire (44 ; 72), qui est fixé sur l'arbre d'entraînement (28 ; 70) de manière excentrée à l'axe médian longitudinal (32) de celui-ci et/ou sur l'élément de coupe (20 ; 68) de manière excentrée à l'axe médian longitudinal (22) de celui-ci, **caractérisé en ce que** le diamètre de l'élément filaire est plus petit que le diamètre de l'arbre d'entraînement (28 ; 70).

2. Instrument selon la revendication 1, **caractérisé en ce que** l'élément filaire (44, 72) est fixé sur le pourtour de l'arbre d'entraînement (28 ; 70) et/ou sur le pourtour de l'élément de coupe (20 ; 68).

3. Instrument selon la revendication 2, **caractérisé en ce que** l'élément filaire (44), dans la zone de la fixation sur l'arbre d'entraînement (28) et/ou dans la zone de la fixation sur l'élément de coupe (20), est coudé par rapport à l'axe médian longitudinal de la tige (12).

4. Instrument selon l'une quelconque des revendications 1 à 3, **caractérisé en ce que** la tige (12) comporte exactement une zone de flexion (26) et **en ce que** l'élément filaire (44) est fixé sans déport périphérique sur l'arbre d'entraînement (28) et sur l'élément de coupe (20).

5. Instrument selon l'une quelconque des revendications 1 à 3, **caractérisé en ce que** la tige (62) comporte deux zones de flexion (64, 66) en sens opposé l'une par rapport à l'autre, et **en ce que** l'élément filaire (72) est fixé sans déport périphérique sur l'arbre d'entraînement (70) et sur l'élément de coupe (72).

6. Instrument selon l'une quelconque des revendications 1 à 3, **caractérisé en ce que** la tige (62) comporte deux zones de flexion (64, 66) en sens opposé l'une par rapport à l'autre, et **en ce que** l'élément filaire (72) est fixé sur l'arbre d'entraînement (70) et sur l'élément de coupe (72) dans une position angulaire déportée de 180° environ l'un par rapport à l'autre.

7. Instrument selon l'une quelconque des revendications 1 à 6, **caractérisé en ce que** l'élément filaire (44 ; 72) est un fil en matériau plein ou un tube, dont le diamètre est plus petit que la moitié du diamètre intérieur de la tige (12 ; 62).

8. Instrument selon l'une quelconque des revendications 1 à 7, **caractérisé en ce que** l'élément filaire (44 ; 72) est réalisé en acier à ressort.

9. Instrument selon l'une quelconque des revendications 1 à 8, **caractérisé en ce que** l'élément filaire (44 ; 72) est fixé par brasage, soudage ou collage sur l'arbre d'entraînement (28 ; 70) et/ou sur l'élément de coupe (20 ; 68).

10. Instrument selon l'une quelconque des revendications 1 à 9, **caractérisé en ce que** l'élément filaire (44 ; 72) est enfiché avec son extrémité proximale (46) dans l'arbre d'entraînement (28 ; 70) et/ou avec son extrémité distale (48) dans l'élément de coupe (20).
